# FASCICULE DE BREVET EUROPEEN

(11) **EP 0 655 247 B1**
(45) Date de publication et mention de la délivrance du brevet: **31.10.2001**
(21) Numéro de dépôt: 94403014.7
(22) Date de dépôt: 21.05.1991
(51) Int. Cl.: A61K 31/44, A61K 31/475

(54) **Utilisation de la vincristine pour l'évaluation de substances au niveau neuronal**
Verwendung von Vincristin zur Auswertung von Wirkstoffen in Zentralnervensystems
Use of vincristine for the evaluation of substances at the neuroral level

(30) Priorité: 22.05.1990 FR 9006399
(43) Date de publication de la demande: 31.05.1995
(62) Demande divisionnaire de: 91401311.5
(73) Titulaire: SANOFI-SYNTHELABO, 75013 Paris (FR)
(72) Inventeur: Coude, François-Xavier, F-31500 Toulouse (FR); Fournier, Jacqueline, F-31830 Plaisance du Touch (FR); Guzzi, Umberto, I-20100 Milano (IT)
(74) Mandataire: Le Roux, Martine

(56) Documents cités:
- EUROPEAN JOURNAL OF PHARMACOLOGY, vol.162, 1989 pages 43 - 50 DI PATRE, P.L. ET AL 'GM1 GANGLIOSIDE COUNTERACTS CHOLINERGIC AND BEHAVIOURAL DEFICITS INDUCED IN THE RAT BY INTRACEREBRAL INJECTION OF VINCRISTINE'
- LIFE SCIENCES, vol.35, no.1, 1984 pages 43 - 51 STEWARD, O. ET AL 'NEUROTOXICITY OF COLCHICINE AND OTHER TUBULIN-BINDING AGENTS: A SELECTIVE VULNERABILITY OF CERTAIN NEURONS TO THE DISRUPTION OF MICROTUBULES'
- BRAIN RESEARCH, vol.486, no.1, 1989 pages 133 - 140 GOLDSCHMIDT, R.B. ET AL 'COMPARISON OF THE NEUROTOXIC EFFECTS OF COLCHICINE, THE VINCA ALKALOIDS, AND OTHER MICROTUBULE POISONS'

## Description

La présente invention concerne une méthode pour le screening pharmacologique de substances actives au niveau neuronal qui comprend :
- la production de lésions neuronales à des animaux non-humains destinés à l'expérimentation pharmacologique par injection dans le septum médian des animaux d'une quantité de vincristine efficace à ce but ;
- l'administration de la substance à tester aux animaux ainsi lésés selon le protocole convenablement choisi ;
- le sacrifice des animaux ; et
- l'évaluation des effets du traitement dans des tests morphologiques et/ou biochimiques.

Selon l'invention, on a mis au point un nouveau modèle expérimental pour l'évaluation in vivo de l'activité neurotrophique/neuroprotectrice de composés test dans des processus de dégénérescence neuronale.

Un modèle expérimental pour ce type d'évaluation a été récemment proposé par Y. Nakagawa et al. (Brain Research, 1987, 408, 57-64).

Dans l'étude qu'ils ont conduite, ils ont mis en évidence la ressemblance entre les modifications neurochimiques et comportamentales, provoquées par infusion d'un agent neurotoxique, notamment de colchicine, dans l'hippocampe et celles qu'on retrouve dans les patients atteints de la maladie d'Alzheimer. Sur la base des résultats publiés par Y. Nakagawa et en tenant compte des évidences bibliographiques du rôle de l'hippocampe dans les phénomènes mnémoniques on a essayé de mettre au point un modèle expérimental de meilleure faisabilité qui soit encore plus proche de la physiopathologie rencontrée dans la maladie d'Alzheimer.

Le modèle qu'on a mis en place répond aux questions suivantes: bonne faisabilité et lésions irréversibles, très spécifiques pour le système cholinergique septohippocampique.

En particulier, on a provoqué des lésions des neurones septaux par injection locale de vincristine en tant qu'inhibiteur de la polymérisation de la tubuline.

Par rapport aux autres produits de ce type (colchicine et vinblastine) et aux différents sites d'injection (intraventriculaire et intrahippocampique) qu'on a testés, on a ainsi obtenu les meilleurs résultats soit en terme de spécificité de blocage de la voie cholinergique septohippocampique soit en terme d'irréversibilité des lésions.

### PROTOCOLES EXPERIMENTAUX

### Méthode de production des lésions

Les animaux (rats mâles, Sprague Dawley d'environ 250 g) sont anesthésiés au pentobarbital (10 mg/kg i.p.), puis placés sur un appareil de stéréotaxie. En accord avec les coordonnées de l'atlas Paxinos et Watson, l'injection dans le spetum médian se fait selon le point de coordonnées suivantes:

| | |
|---|---|
| antériorité | 8,9 |
| latéralité | 0 |
| ventralité | 6,4 |
| (le point 0 correspond au lambda) | |

La vincristine est mise en solution dans un liquide céphalorachidien artificiel (ACSF) ayant la composition suivante:

| | |
|---|---|
| NaCl | 150 mM |
| CaCl₂ | 1,8 mM |
| MgSO₄ | 1,2 mM |
| K₂HPO₄ | 2 mM |
| glucose | 10 mM |
| pH 7,4 | |

à raison de 0,6 µmole de vincristine par ml.

1µl de cette solution (0,6 nmole de vincristine) est injecté localement dans le septum médian en 1 minute.

### Méthode d'évaluation des lésions

- **Evaluation morphologique (observation histoenzymologique de l'AchE)**
   Les animaux sont perfusés par l'aorte avec un mélange fixateur (glutaraldéhyde/parafomaldéhyde) à la vitesse de perfusion de 25 ml/min pendant 5 minutes.
   Le cerveau est prélevé, la fixation poursuivie pendant 1 heure; cette étape est suivie par le lavage et la cryoprotection dans du saccharose à 20% dans un tampon phosphate. Le cerveau est alors coupé au cryostat, les coupes de 30 µm d'épaisseur sont prélevées au niveau du septum et de l'hippocampe et montées sur lame. Les lames sont incubées environ 15 heures dans le mélange suivant:
   200 ml d'une solution stock: 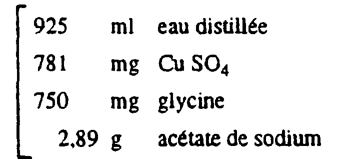 à laquelle on ajoute extemporanément
   230 mg iodure d'acétylthiocholine
   10 mg d'éthopropazine.
   La réaction est ensuite révélée par passage dans du sulfure d'ammonium à 2% et amplifiée par AgNO₃ à 0,25 %.
   Les sites à acétylcholinestérases (en général associés aux synapses cholinergiques) sont révélés sous forme d'un précipité brun.
- **Evaluation biochimique (détermination de l'activité ChAT)**
   La détermination de l'activité ChAT a été conduite selon la méthode de Fonnum (J. Neurochem., 24, 1975, 407-409). Les tissus sont homogénéisés dans un potter (à 4°C).
   Chaque échantillon est ramené à 1 mg de protéines/ml. 10 µl d'homogénat sont incubés en présence de 1,5 mM de choline, 70 µM d'acétylCoA, 30 µM d'acétylCoA marqué au ¹⁴C, en présence d'ésérine 0,15 mM. L'incubation dure 7 minutes 30" à 37°C.
   L'arrêt de la réaction se fait au bain de glace avec 5 ml de tampon phosphate.
   Après addition de 2 ml de tétraphénylboron/acétone et de 5 ml de scintillant, l'acétylcholine marquée au ¹⁴C est comptée au compteur à scintillations. Chaque échantillon est dosé en triplicate. Chaque lot est comparé au lot contrôle correspondant par le test de Student.
- **Evaluation comportamentale**
   Cette étude porte sur des groupes d'animaux réservés à cet effet, les rats étant mis en cycle inversé. Les rats utilisés dans ce cas sont de souche Wistar, lésés selon la même méthode décrite ci-dessus.
- **Mémoire sociale**
   (A.Perio et al., Psychopharmacology, 1989, 97, 262-268). Selon ce test, un rat juvénile est mis dans la cage d'un rat adulte et la durée de la rélation sociale est mesurée en secondes (T1).
   Les animaux sont séparés pendant 15 minutes et le même rat juvénile lui est présenté à nouveau. On mesure de la même façon le temps de contact T2. Dans le cas d'animaux normaux, le souvenir de la première rencontre se traduit par un rapport T2/T1<1. Dans le cas d'une altération de mémoire le rapport est T2/T1≥1.
- **Test du labyrinthe en T**
   Conduit selon la méthode décrite par P. Soubrié et al., J. Pharmacol. (Paris), 1977, 8, 3, 393-403 pour le test du Labyrinthe en Y.
- **Test de la planche à trous**
   (S.E. File et al., Pharmacol. Biochem. Behav., 1985, 22, 941-944).

### Evaluation des lésions

L'administration intraseptale de vincristine entraîne une chute rapide (une semaine après l'injection) et importante (de 60 à 70%) des marqueurs cholinergiques dans l'hippocampe [choline acétyltransférase (ChAT) et acétylcholinestérase (AchE)] ainsi qu'une dégénérescence des neurones du septum médian, qui se révèle maximale deux semaines après l'injection, et qui est associée à la diminution des marqueurs cholinergiques. Cette dégénérescence semble être irréversible, puisqu'on la retrouve trois mois après l'injection de vincristine, et entraîne des altérations fonctionnelles.

Parmi les altérations fonctionnelles consécutives l'administration de vincristine, on a relevé principalement un déficit très important et irréversible des fonctions mnémoniques (test de mémoire sociale). Des évaluations parallèles ont mis en évidence, entre autres, une réduction des capacités d'exploration ("Labyrinthe en T" et "Planche à trous").

### TRAITEMENTS

Dans ce modèle, on a évalué les effets de l'administration du chlorhydrate de 1-[2-(2-naphtyl)éthyl]-4-(3-trifluorométhylphényl)-1,2,3,6-tétrahydropyridine (Composé A) aux animaux lésés en comparaison aux effets obtenus avec le NGF.

Le Composé A est administré, per os, 2 à 3 heures après l'injection de vincristine, en suspension dans la carboxyméthylcellulose 1%, en raison de 10 ml de suspension par kg. Le lot contrôle est traité avec le véhicule. Le traitement est chronique, le produit est administré une fois par jour pendant 11 jours. Le Composé A a été administré à trois doses différentes: 2,5 mg/kg, 5 mg/kg et 10 mg/kg à des groupes de 8 animaux chacun, et 24 heures après l'arrêt du traitement les animaux sont sacrifiés.

Le NGF, au contraire, est administré, par perfusion intraventriculaire en solution dans le ACSF, contenant de l'albumine de rat en raison de 0,01 % et de la gentamycine (1 ml/15 ml), selon la méthode décrite par W. Fisher et al., Nature, 1987, 329 (6134), 65-68. La concentration en NGF de la solution est calculée de telle sorte qu'en fonction du débit de diffusion (0,44 ± 0,02 µl/h) les animaux (7 rats) reçoivent 0,105 µg, 1,05 µg, ou 10,5 µg de NGF en dose totale sur deux semaines de perfusion. Pour le lot contrôle, le NGF est remplacé par une protéine de poids moléculaire équivalent (environ 130.000) ne possédant aucune activité neurotrophique: le cytochrome C. Deux semaines après la lésion et le début du traitement, les animaux sont sacrifiés. Un groupe d'animaux sacrifiés est perfusé pour une évaluation histoenzymologique de l'AChE, les autres animaux sont utilisés pour effectuer un dosage ChAT dans l'hippocampe et le septum.

### RESULTATS

### Evaluation morphologique

Chez les animaux lésés et non traités, on n'observe presque aucun précipité brun tandis que, chez les animaux traités avec 5 mg/kg du Composé A, les bourgeonnements hippocampiques observés donnent une image de la structure proche de celle observée chez un animal normal. On obtient ainsi des résultats similaires à ceux obtenus avec le NGF.

### Evaluation biochimique

La lésion entraîne une chute très significative de l'activité ChAT dans l'hippocampe qui augmente en fonction de la dose du Composé A administré jusqu'à une récupération totale à la dose de 10 mg/kg, comparable à la récupération qu'on obtient dans le groupe d'animaux perfusés avec le NGF. Les résultats sont reportés dans le tableau suivant

**TABLEAU I**

| | Activité ChAT (pmole/mg/mn) |
|---|---|
| Animaux normaux non lésés | 277 ± 13 |
| Contrôles lésés | 115 ± 18 |
| NGF 0,105 µg/rat/2 semaines | 168 ± 27 |
| NGF 1,05 µg/rat/2 semaines | 336 ± 28 |
| NGF 10,5 µg/rat/2 semaines | 293 ± 10 |
| Animaux normaux non lésés | 242 ± 19 |
| Contrôles lésés | 97 ± 18 |
| Composé A 2,5 mg/kg/jour | 164 ± 42 |
| Composé A 5 mg/kg/jour | 204 ± 24 |
| Composé A 10 mg/kg/jour | 242 ± 27 |

Dans le septum, la lésion entraîne une chute de l'activité ChAT dont la récupération est en fonction de la dose du Composé A administré. Dans le cas d'animaux perfusés avec le NGF les résultats ne sont pas significatifs; ceci peut être dû au fait qu'à la nécrose septale associée à l'injection de vincristine se surajoute une nécrose supplémentaire consécutive à la canulation dans un ventricule proche du septum.

### Evaluation comportamentale

- **Mémoire sociale**
   Une fois lésés avec la vincristine, les animaux présentent des altérations de la mémoire sociale qui semblent irréversibles (elles sont encore présentes 50 jours après l'injection de vincristine). Le Composé A a été essayé à la dose de 10 mg/kg per os en comparaison avec le NGF à la dose de 10,5 µg et on a effectué ce test au 7ème jour de la lésion. Dans les deux cas, on a observé un effet protecteur très important en obtenant un rapport T2/T1 compris entre 0,6 et 0,7.
- **Labyrinthe en T**
   Les valeurs obtenues chez les animaux lésés et non traités sont significatives d'une altération des capacités d'exploration qui par contre sont récupérées à un niveau normal par traitement avec le Composé A à la dose de 10 mg/kg. Ce test a été effectué en aveugle, 7 jours après l'arrêt du traitement.
- **Planche à trous**
   Dans le lot contrôle, la majorité (6 rats) des animaux ont perdu toute capacité d'exploration, seuls deux rats montrent une hyperactivité exploratoire. Le traitement avec le Composé A à la dose de 10 mg/kg apporte une normalisation du comportement exploratoire en se référant à des valeurs moyennes d'animaux normaux. Ce test a été effectué, également en aveugle, 11 jours après l'arrêt des traitements.

## Revendications

1. Une méthode pour le screening pharmacologique de substances actives au niveau neuronal qui comprend :
- la production de lésions neuronales à des animaux non-humains destinés à l'expérimentation pharmacologique par injection dans le septum médian des animaux d'une quantité de vincristine efficace à ce but ;
- l'administration de la substance à tester aux animaux ainsi lésés selon le protocole convenablement choisi ;
- le sacrifice des animaux ; et
- l'évaluation des effets du traitement dans des tests morphologiques et/ou biochimiques.

## Patentansprüche

1. Verfahren zum pharmakologischen Screenen von im Nervenzellenbereich aktiven Substanzen, umfassend:
- die Herbeiführung von Schädigungen an Nervenzellen von pharmakologischen Versuchstieren durch Injizieren einer für diesen Zweck wirksamen Menge von Vincristin in die mittlere Scheidewand der Tiere;
- die Verabreichung der Testsubstanz an die so geschädigten Tiere nach dem entsprechend gewählten Protokoll;
- die Tötung der Tiere; und
- die Auswertung der Auswirkungen der Behandlung in morphologischen und/oder biochemischen Tests.

## Claims

1. A method for the pharmacological screening of neuronally active substances which comprises:
- producing neuronal lesions to non-human animals intended for pharmacological experimentation by injection in the medial septum of the animals of an amount of vincristine effective to this purpose;
- administering the substance to be tested to the lesioned animals according to the suitably selected schedule ;
- sacrificing the animals ;
and
- evaluating the effects of the treatment by means of morphological and/or biochemical assays.
